# EUROPÄISCHE PATENTSCHRIFT

(11) **EP 0 586 872 B1**
(45) Veröffentlichungstag und Bekanntmachung des Hinweises auf die Patenterteilung: **02.10.1996**
(21) Anmeldenummer: 93112289.9
(22) Anmeldetag: 30.07.1993
(51) Int. Cl.: C07K 1/107, C12N 9/16, C12N 15/55, C12N 15/70

(54) **Verfahren zur Gewinnung rekombinanter, biologisch aktiver, eukaryontischer alkalischer Phosphatase**
Method for the production of recombinant, biologically active eukaryiotic alkaline phosphatase
Procédé de production de la phosphatase alcaline eukaryotique recombinante, biologiquement active

(30) Priorität: 31.07.1992 DE 4225427
(43) Veröffentlichungstag der Anmeldung: 16.03.1994
(73) Patentinhaber: BOEHRINGER MANNHEIM GMBH, D-68305 Mannheim-Waldhof (DE)
(72) Erfinder: Michaelis, Uwe, Dr., D-82362 Weilheim (DE); Rudolph, Rainer, Prof., D-82362 Weilheim (DE); Jarsch, Michael, Dr., D-83670 Bad Heilbrunn (DE); Kopetzki, Erhard, Dr., D-82377 Penzberg (DE); Burtscher, Helmut, Dr., D-82392 Habach (DE); Schumacher, Günther, Dr., D-82347 Bernried (DE)
(74) Vertreter: Böhm, Brigitte, Dipl.-Chem. Dr.

(56) Entgegenhaltungen:
- EP-A- 0 151 320
- HERMANN, R. 'EPO Applied Technology Series vol. 12: Protein Folding' 1993 , EUROPEAN PATENT OFFICE , NETHERLANDS
- ENZYME MICROB. TECHNOL. Bd. 1, 1979, Seiten 74 - 81 TORCHILIN, V.P.and MARTINEK K, Seite 74
- BIO/TECHNOLOGY Bd. 8, 1990, Seiten 1274 - 1278 CLELAND, J.L. & WANG, D.I.C 'cosolvent assisted protein refolding'
- TIMASHEFF, S.N. ET AL. (FRANKS & MATHIAS EDS.) 'The role of solvation in protein structure stabilisation and unfolding *' 1982 , WILEY & SONS
- CHEMICAL ABSTRACTS, no. 69, 1968, Columbus, Ohio, US; abstract no. 41426m, BUTTERWORTH, P.J. 'The reversible inactivation of pig kidney alkaline phosphatase at low pH *' Seite 3871 ;
- CHEMICAL ABSTRACTS, vol. 91, 1979, Columbus, Ohio, US; abstract no. 89776g, LINDEN, G. 'Biochemical study of some aspects of milk alkaline phosphatase reactivation' Seite 89769 ;

### SEQUENZPROTOKOLL

(1) ALLGEMEINE INFORMATION:
   (i) ANMELDER:
      (A) NAME: BOEBRINGER MANNHEIM GMBH
      (B) STRASSE: Sandhofer Str. 116
      (C) ORT: Mannheim
      (D) BUNDESLAND: BW
      (E) LAND: Germany
      (F) POSTLEITZAHL: D-6800
      (G) TELEPHON: 0621/759-3197
      (H) TELEFAX: 0621/759-4457
   (ii) ANMELDETITEL: Verfahren zur Gewinnung rekombinanter, biologisch aktiver, eukaryontischer alkalischer Phosphatase
   (iii) ANZAHL DER SEQUENZEN: 3
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
   (vi) FRÜHERE ANMELDEDATEN:
      (A) ANMELDENUMMER: DE P 42 25 427.2
      (B) ANMELDEDATUM: 31-JUL-1992
(2) INFORMATION ZU SEQ ID NO: 1:
   (i) SEQUENZ CHARAKTERISTIKA:
      (A) LÄNGE: 33 Basenpaare
      (B) ART: Nukleinsäure
      (C) STRANGFORM: Einzel
      (D) TOPOLOGIE: linear 
(xi) SEQUENZBESCHREIBUNG: SEQ ID NO: 1:

GCGCGTCGAC TCATGATCAT CCCAGTTGAG GAG 33
(2) INFORMATION ZU SEQ ID NO: 2:
   (i) SEQUENZ CHARAKTERISTIKA:
      (A) LÄNGE: 16 Basenpaare
      (B) ART: Nukleinsäure
      (C) STRANGFORM: Einzel
      (D) TOPOLOGIE: linear 
(xi) SEQUENZBESCHREIBUNG: SEQ ID NO: 2:

CATCCCATCG CCCAGG 16
(2) INFORMATION ZU SEQ ID NO: 3:
   (i) SEQUENZ CHARAKTERISTIKA:
      (A) LÄNGE: 30 Basenpaare
      (B) ART: Nukleinsäure
      (C) STRANGFORM: Einzel
      (D) TOPOLOGIE: linear

(xi) SEQUENZBESCHREIBUNG: SEQ ID NO: 3:

CAATTAAGCT TTTATCAGTC GGTGGTGCCG 30

## Beschreibung

Die vorliegende Erfindung betrifft ein Verfahren zur Gewinnung biologisch aktiver, rekombinanter, eukaryontischer alkalischer Phosphatase durch Expression in Prokaryonten.

Alkalische Phosphatasen sind in der Natur weitverbreitete Metalloenzyme, die eine Hydrolyse von Phosphatesterbindungen bei alkalischen pH-Werten katalysieren. Sie sind z.B. als Tumor-assoziierte Proteine (Fröhlander, N.S. Millan, J.L. (1991), In vivo 5, 483-488; McComb et al. (1979), Alkaline Phosphatases, Plenum Press) seit langem beschrieben. Ihre eigentliche Funktion ist jedoch noch weitgehend unbekannt. Alkalische Phosphatasen finden in der biologischen Forschung ebenso wie in der Gentechnologie und in der medizinischen Diagnostik, z.B. als wichtige Markerenzyme für Enzym-Immunassays, vielseitige Verwendung. Eukaryontische alkalische Phosphatasen haben, im Gegensatz zu den beschriebenen prokaryontischen Enzymen, meist eine höhere spezifische Aktivität (McComb et al. (1979), Alkaline Phosphatases, Plenum Press). Die Aktivitäten der alkalischen Phosphatasen bei Säugetieren sind zudem gewebsspezifisch und kommen z.B. im intestinalen Epithel (Darm), in der Placenta, in der Niere, den Knochen und in der Leber vor (Meyer-Sabellek et al. (1988), Journal of Chromatography 429, 419-444).

Eine kostengünstige rekombinante Herstellung von eukaryontischen alkalischen Phosphatasen mit konstanter Qualität ist daher, auch im Hinblick auf eine Verringerung der Infektionsgefahr (HIV, BSE) bei ihrer Isolierung aus Geweben sowie ebenso aufgrund des Problems der Gewinnung von heterogenen Enzymgemischen bei der Isolierung aus Geweben, von großem Interesse.

Bei der heterologen Expression von eukaryontischen Proteinen in Prokaryonten entstehen häufig schwer lösliche, biologisch inaktive Proteinaggregate (Inclusion Bodies). Diese können in den meisten Fällen mit an sich bekannten Methoden (Jaenicke, R. (1979) FEBS Letters, Vol. 52, 187-198; Rudolph R. (1990), Modern Methods in Protein and Nucleic Acid Research, 149-171) wieder in ihre biologisch aktive Form zurückgeführt werden. Dabei werden die in "Inclusion Bodies" vorliegenden Proteine durch Zugabe von starken Denaturierungsmitteln, wie z.B. Harnstoff, Guanidinhydrochlorid, oder durch Zugabe von stark sauren Agenzien, beispielsweise Glycin/Phosphorsäuremischungen, unter reduzierenden Bedingungen solubilisiert und anschließend renaturiert.

Im Stand der Technik werden Verfahren zur Renaturierung von verschiedenen denaturierten Proteinen beschrieben (z.B. DE 35 37 708 A1, DE 38 35 350 A1, EP-A 0 241 022).

In EP-A 0 114 506 beispielsweise ist ein Verfahren zur Reinigung und Reaktivierung heterolog exprimierter Proteine offenbart. Dabei werden die in "Inclusion Bodies" vorliegenden Proteine mit einem stark denaturierenden Mittel gelöst und anschließend in ein schwächer denaturierendes Lösungsmittel überführt, wo sie zur Ausbildung der Disulfidbrücken oxidierenden Bedingungen unterworfen werden, oder vor Überführung in ein schwächer denaturierendes Lösungsmittel sulfoniert und anschließend, in Gegenwart des schwächer denaturierenden Lösungsmittels, durch Umsatz mit einem Sulfhydrylreagenzes in seiner reduzierten und oxidierten Form unter Ausbildung von S-S-Bindungen renaturiert werden.

Obschon eine Reihe von eukaryontischen alkalischen Phosphatasen kloniert wurden (Kam et al. (1985), Proc. Natl. Acad. Sci., USA 82, 8715-8719; Millan J.L. (1986), J. Biol. Chem. 261, 3112-3115; Henthorn et al. (1987), Proc. Natl. Acad. Sci., USA 84, 1234-1238; Hsu, H.H.T. & Anderson, H.C. (1989), Int. J. Biochem. 21, 847-851; Millan, J.L. (1988), Anticancer Research 8, 995-1004; Harris, H. (1989), Clinica Chimica Acta 186, 133-150; Smith, A.F. (1989), Clin. Chem. Enzym. Comms 2, 1-22; Millan J.L., (1990), Isozymes: Structure, Function and Use in Biology and Medicine, 453-475, Wiley-Liss Inc.), gelang bisher eine Expression in Prokaryonten mit erfolgreicher Renaturierung des Enzyms in seine biologisch aktive Form nicht.

Aufgabe der vorliegenden Erfindung ist daher die Bereitstellung eines Verfahrens zur Renaturierung eukaryontischer alkalischer Phosphatasen nach Expression in Prokaryonten.

Diese Aufgabe wird erfindungsgemäß dadurch gelöst, daß eine für eine eukaryontische, alkalische Phosphatase kodierende DNA-Sequenz in einer prokaryontischen Wirtszelle exprimiert wird und das Expressionsprodukt in aktiver Form durch Zellaufschluß, Solubilisierung unter denaturierenden Bedingungen und anschließender Renaturierung gewonnen wird, wobei der Renaturierungsschritt in Gegenwart eines oder mehrerer Stabilisierungsmittel durchgeführt wird, die aus synthetischen Polymeren, Mono-, Oligo- und Polysacchariden, Polyalkoholen und Salzen ausgewählt werden und in einer die Renaturierungseffizienz verbessernden Konzentration vorliegen.

Durch das erfindungsgemäße Verfahren wird es überraschenderweise ermöglicht, alkalische Phosphatasen nach Expression in Prokaryonten in biologisch aktiver Form zu erhalten, wobei es insbesondere bemerkenswert ist, daß die Renaturierung heterolog exprimierter alkalischer Phosphatasen, im Gegensatz zu den bereits bekannten Verfahren, nur in Gegenwart eines oder mehrerer Stabilisierungsmittel erfolgreich durchzuführen ist. Besonders bevorzugt wird das Verfahren mit placentaler alkalischer Phosphatase durchgeführt, doch auch andere Phosphatasen wie Phosphatase aus Kälberdarm etc. haben sich als geeignet erwiesen.

Als Stabilisierungsmittel im Sinne der Erfindung können Agenzien, die eine Stabilisierung von Proteinen in Lösung bewirken, d.h. synthetische Polymere, Mono-, Oligo- und Polysaccharide, Polyalkohole, Salze u.a., verwendet werden (Gupta, M.N. (1991), Biotechnology and Applied Biochemistry 14, 1-11; Gray, C.J. (1988), Biocatalysis 1, 187-196; Busby, T.F. & Ingham, K.C. (1984), Biochem. Biophys., Acta 799, 483-488). Dabei werden erfindungsgemäß Sulfatsalze, Kohlenhydrate oder Polyalkohole entweder alleine oder in Kombination mit einander bevorzugt.

In einer bevorzugten Ausführungsform des erfindungsgemäßen Verfahrens werden Natriumsulfat oder Kaliumsulfat oder Ammoniumsulfat oder Mischungen derselben als Stabilisierungsmittel verwendet. Natriumsulfat wird dabei vorzugsweise in einer Menge von 0,3 bis 1 mol/l verwendet, bevorzugter in einer Menge von 0,3 bis 0,6 mol/l. Kaliumsulfat wird vorzugsweise in einer Menge von 0,1 bis 0,6 mol/l, besonders bevorzugt in einer Menge von 0,1 bis 0,45 mol/l verwendet. Ammoniumsulfat wird bevorzugt in einer Menge von 0,3 bis 1 mol/l verwendet.

In einer weiteren bevorzugten Ausführungsform werden als Stabilisierungsmittel Kohlenhydrate verwendet. Vorzugsweise werden dabei die Kohlenhydrate in einer Menge von 5 bis 50 Gew./Vol.-%, bezogen auf das Volumen des Renaturierungsansatzes, eingesetzt. Als Kohlenhydrate werden vorzugsweise Pentosen, wie z.B. Arabinose, Hexosen, wie z.B. Galactose, Glucose oder Fructose, verschiedene Disaccharide, wie z.B. Lactose, Maltose oder Saccharose oder Gemische von Pentosen, Hexosen oder/und Disacchariden eingesetzt.

Ebenso können in einer weiteren bevorzugten Ausführungsform verschiedene Polyalkohole in einer Menge von 5 bis 50 Gew./Vol.-%, bezogen auf das Volumen des Renaturierunsansatzes, wie z.B. Sorbitol, Glycerin, Erythritol, Inositol, Ethylenglycol oder Gemische davon verwendet werden.

In einer besonders bevorzugten Ausführungsform der vorliegenden Erfindung können Gemische von Kohlenhydraten, Polyalkoholen oder/und Sulfatsalzen zur Renaturierung von eukaryontischen alkalischen Phosphatasen eingesetzt werden. Dabei wird überraschenderweise ein synergistischer Effekt beobachtet, der eine Effizienzsteigerung in der Renaturierung bewirkt, die über den additiven Wert der einzelnen Komponenten hinausgeht.

Durch die Beschreibung der verwendeten Standardrenaturierungspuffer in den Beispielen, zu dem erfindungsgemäß die jeweiligen Stabilisierungsmittel zugesetzt werden, soll jedoch nicht ausgeschlossen werden, daß Pufferlösungen mit anderen Zusammensetzungen zur Verwendung in dem erfindungsgemäßen Verfahren geeignet sind.

Die heterologe Expression einer klonierten DNA-Sequenz in einer prokaryontischen Wirtszelle ist aus dem Stand der Technik bekannt. Die Einführung der DNA-Sequenz in die Wirtszelle kann beispielsweise durch Transformation mit einem Vektor erfolgen, der mindestens eine Kopie der DNA-Sequenz enthält. Hierbei sind sowohl extrachromosomale Vektoren (z.B. Plasmide) als auch Integrationsvektoren (z.B. Lambda-Vektoren) geeignet, wobei jedoch Plasmide bevorzugt sind. Die prokaryontische Wirtszelle ist vorzugsweise ein gram-negative Wirtszelle, besonders bevorzugt eine E.coli-Zelle. Hinsichtlich der verschiedenen Techniken zur Klonierung von Genen und der Transformation von Wirtszellen wird auf Sambrook et al. (Molecular Cloning. A Laboratory Manual (1989), Cold Spring Harbor Laboratory Press) verwiesen.

Die Kultivierung der transformieren Wirtszelle bei dem erfindungsgemäßen Verfahren zur Gewinnung von rekombinanter, biologisch aktiver, eukaryontischer, alkalischer Phosphatase findet unter solchen Bedingungen statt, die für die Expression der DNA-Sequenz förderlich sind. In einer bevorzugten Ausführungsform ist die DNA-Sequenz operativ mit einem regulierbaren Promotor verknüpft, der durch Zugabe eines Induktors oder durch Temperaturerhöhung induziert werden kann. Weiterhin kann es bevorzugt sein, daß neben dem das Phosphatasegen enthaltenden Vektor weitere, die Expression verbessernde Hilfsvektoren in der Wirtszelle vorliegen (z.B. pUBS 500). Ein besonders bevorzugter Vektor zur Gewinnung rekombinanter placentaler alkalischer Phosphatase ist das Plasmid pPLAP (DSM 7094).

Der Zellaufschluß nach der Expression kann durch alle hierfür üblichen Methoden ausgeführt werden, z.B. mittels Ultraschall, Hochdruckdispersion oder Lysozym. Er wird vorzugsweise in einer zur Einstellung eines neutralen bis schwach sauren pH-Wertes geeigneten Pufferlösung durchgeführt, wie z.B. 0,1 mol/l Tris/HCl. Die aufgeschlossenen Zellen werden dann vorzugsweise mittels Zentrifugation oder Filtration in eine lösliche und eine unlösliche Fraktion getrennt. Die alkalische Phosphatase befindet sich dabei im allgemeinen in Form von "Inclusion Bodies" in der unlöslichen Fraktion bzw. dem Pellet. Nach Waschen des Pellets mit Agenzien, die die in Form von "Inclusion Bodies" vorliegenden Proteine der alkalischen Phosphatasen nicht stören, Verunreinigungen, wie z.B. fremde Proteine, jedoch möglichst lösen, wird das Pellet nach bekannten Methoden einer Solubilisierungsprozedur (Solubilisierung, Reduktion) unterworfen. Die Solubilisierung erfolgt vorzugsweise im alkalischen pH-Bereich, insbesondere bei pH 8 in Gegenwart von reduzierenden Agenzien und hohen Konzentrationen denaturierender Mittel, vorzugsweise 8 mol/l Harnstoff. Die Solubilisierung kann z.B. bei Raumtemperatur für eine Zeit von mindestens 2 Stunden erfolgen. Der pH-Wert wird bevorzugt nach erfolgter Solubilisierung auf Werte von 3 bis 4 mittels Zugabe von HCl eingestellt und in einem weiteren Reinigungsschritt werden nach bekannten Methoden die unlöslichen Stoffe abgetrennt. Das verwendete Reduktionsmittel wird anschließend vorzugsweise mittels Dialyse gegen das Denaturierungsmittel entfernt.

Das erfindungsgemäße Verfahren ist dadurch gekennzeichnet, daß der Renaturierungsschritt in Gegenwart von einem oder mehreren der oben genannten Stabilisierungsmittel durchgeführt wird. Dabei ist es bevorzugt, daß die Renaturierung im wesentlichen in Abwesenheit (≤ 50 mmol/l) von chaotropen, d.h. denaturierenden Agenzien, wie etwa Guanidiniumhydrochlorid oder Harnstoff, durchgeführt wird. Es wurde jedoch gefunden, daß auch bei höheren, aber nicht denaturierend wirkenden Konzentrationen an chaotropen Agenzien, (z.B. bis 800 mmol/l Harnstoff) die erfindungsgemäße Renaturierung von alkalischer Phosphatase bei gleichzeitiger Anwesenheit von oben genannten Stabilisierungsmitteln möglich ist, wenngleich eine geringere Renaturierungseffizienz beobachtet wird.

Der Renaturierungsschritt kann nach allen bekannten und üblichen Prozeduren durchgeführt werden, vorausgesetzt daß ein oder mehrere Stabilisierungsmittel vorhanden sind. So ist es möglich, die aus EP-A 0 241 022 bekannte Methode der Pulsrenaturierung und gleichzeitig damit entweder das in DE 3 835 350 A1 offenbarte Verfahren über die gemischten Disulfide oder die direkte Umsetzung mit einem Disulfid und einem Mercaptan in dem erfindungsgemäßen Verfahren einzusetzen. Dabei kann es aber zweckmäßig sein, einzelne oder alle Verfahrensschritte unter Berücksichtigung der hier erläuterten Verfahrensausgestaltung durchzuführen.

Dabei wird die Renaturierung vorzugsweise bei 10 bis 40°C, besonders bevorzugt bei 20°C durchgeführt. Ein bevorzugt verwendeter Renaturierungspuffer enthält Tris-HCl-Puffer in einer Menge von 0,1 bis 0,3 mol/l und weist pH-Werte von 6 bis 10, vorzugsweise von 7 bis 9, in der bevorzugtesten Ausführungsform einen pH-Wert von etwa 8 auf, und enthält Magnesiumionen und Zinkionen in Mengen von 0,1 bis 200 mmol/l (Magnesiumchlorid) bzw. 0,01 bis 0,5 mmol/l (Zinkchlorid). Vorzugsweise ist die Menge an Magnesiumionen im Renaturierungsansatz 10 mmol/l und die an Zinkionen 0,1 mmol/l. Der Renaturierungsansatz enthält weiterhin Vorzugsweise Sulfhydrylreagenzien in einer Konzentration von vorzugsweise 0,1 bis 10 mmol/l, besonders bevorzugt von 2 bis 5 mmol/l. Ein im erfindungsgemäßen Verfahren bevorzugtes Sulfhydrylreagenz ist Glutathion in seiner reduzierten oder/und seiner oxidierten Form.

Ein weiterer Gegenstand der Erfindung ist eine rekombinante eukaryontische, nicht glykosylierte, alkalische Phosphatase, die nach dem erfindungsgemäßen Verfahren erhältlich ist. Die spezifische Aktivität der rekombinanten alkalischen Phosphatase liegt nach einer ersten Grobreinigung bei ca. 1000 U/mg. Durch weitere Reinigungsschritte kann die spezifische Aktivität um ca. den Faktor 2-3 erhöht werden.

Die folgenden Beispiele erläutern in Verbindung mit den Tabellen 1 bis 11 und den Sequenzprotokollen SEQ ID NO.1 bis 3 die Erfindung weiter.
- Tab. 1: zeigt die Abhängigkeit der Renaturierungseffizienz von der Na₂SO₄-Konzentration,
- Tab. 2: zeigt die Abhängigkeit der Renaturierungseffizienz von der K₂SO₄- bzw. (NH₄)₂SO₄-Konzentration,
- Tab. 3: zeigt die Abhängigkeit der Renaturierungseffizienz von Galactose, Glucose und Fructose, Lactose, Maltose, Saccharose, Sorbitol, Glycerin, Ethylenglykol und Arabinose,
- Tab. 4: zeigt den Effekt von Glycerin und K₂SO₄ auf die Renaturierungseffizienz,
- Tab. 5: zeigt die Abhängigkeit der Renaturierungseffizienz vom pH-Wert,
- Tab. 6: zeigt die Abhängigkeit der Renaturierungseffizienz von der GSH-Konzentration,
- Tab. 7: zeigt die Abhängigkeit der Renaturierungseffizienz von der GSSG-Konzentration,
- Tab. 8: zeigt die Abhängigkeit der Renaturierungseffizienz von der GSH- und GSSG-Konzentration bei einem äquimolaren GSH/GSSG-Verhältnis,
- Tab. 9: zeigt die Abhängigkeit der Renaturierungseffizienz von der ZnCl₂-Konzentration,
- Tab. 10: zeigt die Abhängigkeit der Renaturierungseffizienz von der MgCl₂-Konzentration und
- Tab. 11: zeigt den Einfluß der Temperatur auf die Renaturierungseffizienz.
- SEQ ID NO.1: zeigt den zur Amplifizierung des PLAP-Strukturgens verwendeten Primer (1),
- SEQ ID NO.2: zeigt den zur Amplifizierung des PLAP-Strukturgens verwendeten Primer (2),
- SEQ ID NO.3: zeigt den zur Amplifizierung des PLAP-Strukturgens verwendeten Primer (3).

Der in den Beispielen verwendete Mikroorganismus E.coli RM82 wurde am 13.07.1989 bei der Deutschen Sammlung für Mikroorganismen und Zellkulturen GmbH (DSM), Mascheroder Weg 1b, D-3300 Braunschweig unter der Hinterlegungsnummer DSM 5445 hinterlegt. Das Plasmidgemisch pPLAP/pUBS500 wurde bei der gleichen Hinterlegungsstelle, am 3. Juni 1992 unter der Hinterlegungsnummer DSM 7094 hinterlegt.

Verwendete Abkürzungen:
- AP:: Alkalische Phosphatase
- DTE:: Dithioerythritol
- GSH:: Reduziertes Glutathion
- GSSG:: Oxidiertes Glutathion
- IBs:: "Inclusion Bodies"
- Mucosa-AP:: Alkalische Phosphatase aus Kälberdarm
- PLAP:: Placentale alkalische Phosphatase
- RT:: Raumtemperatur
- ÜN:: Über Nacht

### Beispiel 1

### Konstruktion des Expressionsplasmids pPLAP

### 1.1 Subklonierung des PLAP Gens

Das PLAP Gen wurde als ca. 2 kBp langes EcoRI/KpnI-Fragment aus dem Lambda-gt11-PLAP Klon (Millan, J.L. (1986), J.Biol.Chem. 261, 3112-3115) isoliert und in den mit EcoRI und KpnI verdauten ca. 2,85 kBp langen pGEM3 Vektor der Firma Promega ligiert.

### 1.2 Konstruktion des E. coli Expressionsvektors pD-NX

Das Plasmid pD-NX ist ein Derivat des Plasmids pQE-6 (pDS56/RBSII,Nco1) der Firma Diagen (Düsseldorf), in dem das promotorlose Chloramphenicol- Acetyltransferase Gen (CAT) entfernt wurde.

Dazu wurde das Plasmid pDS56/RBSII,Ncol mit den Restriktionsendonukleasen NheI und XbaI verdaut, das ca. 2,6 kBp lange NheI/XbaI-Vektorfragment isoliert und die kompatiblen Enden der NheI und XbaI Schnittstelle durch Ligation verknüpft (Konstruktion: pD-NX).

### 1.3 Konstruktion des E. coli Expressionsplasmids pPLAP

Mittels PCR-Technik (Mullis und Faloona (1987), Meth. Enzymol. 155, 335-350) wurde das PLAP Strukturgen von Basenpaarposition 105 - 1556 (Nummerierung entsprechend der Publikation von Millan, 1986) in 2 Teilreaktionen amplifiziert. Die PCR Primer wurden so gewählt, daß das PLAP Strukturgen nach PCR Reaktion von geeigneten Restriktionsendonukleaseschnittstellen flankiert wird (5'-Ende: BspHI und 3'-Ende: HindIII). Danach wurde das PLAP Strukturgen in einer Dreifragmentligation aus den beiden PCR Fragmenten zusammengesetzt und in den E. coli Vektor pD-NX insertiert.

Zur Amplifikation des N-terminalen PLAP Strukturgens wurde das folgende Primerpaar (1) und (2) (siehe SEQ ID NO. 1 und SEQ ID NO. 2) und Plasmid pGEM3-PLAP als Template DNA verwendet.

Primer (1): 5'-GCGCGTCGACTCATGATCATCCCAGTTGAGGAG-3'
BspHI

Primer (2): 5′-CATCCCATCGCCCAGG-3′

Zur Amplifikation des restlichen PLAP Strukturgens wurde das folgende Primerpaar (siehe SEQ ID NO. 1 und SEQ ID NO. 3) verwendet und Plasmid pGEM3-PLAP als Template DNA.

Primer (1): 5'-GCGCGTCGACTCATGATCATCCCAGTTGAGGAG-3'

Primer (3): 5'-CAATTAAGCTTTTATCAGTCGGTGGTGCCG-3'

Das ca. 150 Bp lange PCR Produkt der ersten Reaktion wurde mit BspHI und PstI nachgespalten und das ca. 90 Bp lange BspHII/PstI-Fragment isoliert. Das ca. 1,48 kBp lange PCR Produkt der zweiten Reaktion wurde mit PstI und HindIII verdaut und das ca. 1,39 kBp lange PstI/HindIII-Fragment isoliert. Danach wurden die PCR Fragmente in das ca. 2,55 kBp lange NcoI/HindIII-pD-NX Vektorfragment ligiert (Dreifragmentligation). Das gewünschte Plasmid pPLAP wurde durch Restriktionskartierung identifiziert und partiell sequenziert (Klonierungsübergänge).

### Beispiel 2

### Expression von PLAP in E. coli

Zur Expression der PLAP wurde der E. coli K12 Stamm RM82 (DSM 5445, eine Methionin Revertante von ED 8654, (Murray, N.E. (1977), Mol. Gen. Genet. 150, 53-61) mit dem PLAP Expressionsplasmid pPLAP (Ampicillin-Resistenz) und dem lacI-Repressorplasmid pUBS500 (Kanamycin-Resistenz, Herstellung und Beschreibung siehe: EP-A 0 373 365) transformiert.

Die RM82/pUBS500/pPLAP-Zellen wurden in DYT-Medium (1% (w/v) Hefeextrakt, 1% (w/v) Bacto Tryptone, Difco, und 0,5% NaCl) mit 50 mg/l Ampicillin und 50 mg/l Kanamycin bis zu einer optischen Dichte bei 550 nm von 0,6 - 0,9 angezogen und anschließend mit IPTG (1 - 5 mmol/l Endkonzentration) induziert. Nach einer Induktionsphase von 4 - 8 Stunden wurden die Zellen durch Zentrifugation geerntet, mit 25 mmol/l Tris-HCl-Puffer, pH 7,5, gewaschen und bis zur Weiterverarbeitung bei -20°C gelagert.

### Beispiel 3

### PLAP Expressionsanalyse in E. coli

Das Zellpellet aus 1 ml abzentrifugiertem Anzuchtmedium (RM82/pUBS500/pPLAP-Zellen) wurde in 0,25 ml 10 mmol/l Phosphatpuffer, pH 6,8, und 1 mmol/l EDTA resuspendiert und die Zellen durch Ultraschallbehandlung aufgeschlossen. Nach Zentrifugation wurde der Überstand mit 1/5 Volumen 5xSDS-Probenpuffer (1xSDS-Probenpuffer: 50 mmol/l Tris-HCl, pH 6,8, 1% SDS, 1% Mercaptoethanol, 10% Glycerin, 0,001% Bromphenolblau) versetzt. Die unlösliche Zelltrümmerfraktion wurde in 0,3 ml 1xSDS-Probenpuffer mit 6 - 8 mol/l Harnstoff resuspendiert, die Proben 5 Minuten bei 95°C inkubiert und zentrifugiert. Danach wurden die Proteine durch SDS- Polyacrylamid Gelelektrophorese (PAGE) aufgetrennt (Laemmli, U.K. (1970), Nature 227, 680-685)und mit Coomassie Brilliant Blue R Farbstoff angefärbt.

Zudem wurden die elektrophoretisch aufgetrennten Proteine auf Nitrocellulosefilter transferiert, fixiert (Towbin, H. (1979), Proc. Natl., Acad. Sci. USA 76, 4350) und die PLAP immunreaktiven Proteine mit einem spezifischen Anti-PLAP Antiserum detektiert.

Das in E. coli synthetisierte PLAP Protein war homogen und wurde ausschließlich in der unlöslichen Zelltrümmerfraktion gefunden (IB's). Durch SDS-PAGE und Westernblotanalyse konnten keine verküzten PLAP Fragmente nachgewiesen werden. Der Anteil der PLAP an dem Gesamtprotein der IBs betrug ca. 30 - 50%.

### Beispiel 4

### Präparation der PLAP-IBs

5 g (Naßgewicht) E. coli RM82/pUBS500/pPLAP-Zellen wurden in 25 ml 0,1 mol/l Tris-HCl, pH 7,0, bei 4°C mit dem Ultraturrax (10.000 rpm) homogenisiert. Nach Zugabe von 7,5 mg Lysozym (1,5 mg Lysozym pro g Zellmaterial) und kurzem Mixen mit dem Ultraturrax wurde der Ansatz für 30 Minuten bei 4°C inkubiert. Anschließend wurden die Zellen mechanisch mittels Hochdruckdispersion vollständig aufgeschlossen. Durch Zugabe von MgCl₂ (ad 2 mmol/l) und DNAse (ad 1 mg/100 ml) wurde die DNA bei 25 °C in 30 Minuten verdaut. Anschließend wurde zur Aufschlußlösung ein halbes Volumenteil 60 mmol/l EDTA, 6% Triton X100, 1,5 mol/l NaCl, pH 7,0 zugegeben und weitere 30 Minuten bei 4 °C inkubiert. Danach wurden die unlöslichen Bestandteile (Zelltrümmer und IBs) durch Zentrifugation mit einer Sorvall-Zentrifuge sedimentiert (SS34/20.000 rpm/10 min/4°C). Das Pellet wurde in 40 ml 0,1 mol/l Tris-HCl, 20 mmol/l EDTA, pH 6,5 mit dem Ultraturrax resuspendiert und erneut wie oben zentrifugiert. Das Pellet dieser Zentrifugation repräsentierte die PLAP-IBs.

### Solubilisierung der rekombinanten PLAP

25 mg der PLAP-IBs (Feuchtgewicht) wurden in 3 ml 0,1 mol/l Tris-HCl, 8 mol/l Harnstoff, pH 8,0 (RT) in Gegenwart von 100 mmol/l DTE für mindestens 2 h bei RT suspendiert. Anschließend wurde der pH-Wert des Solubilisierungsansatzes mit HCl auf 3,0 - 4,0 eingestellt und unlösliche Bestandteile durch Zentrifugation abgetrennt. Vollständige Entfernung des Reduktionsmittels DTE erfolgte durch Dialyse des Überstandes gegen 8 mol/l Harnstoff, 5 mmol/l HCl, pH ca. 4,0 für 2 x 2 h bei RT, 1 x ÜN bei 4°C und erneut 1 x 2 h bei RT (Dialysepuffer jeweils ca. 500 ml). Die Proteinkonzentration der Solubilisierungssansätze betrug ca. 2,5 mg/ml. Aliquots des jeweiligen Solubilisierungsansatzes konnten bei Bedarf für mindestens zwei Wochen bei -70 °C gelagert werden.

### Beispiel 5

### Renaturierung der Alkalischen Phosphatase

Die Renaturierung der AP wurde durch 1:200-Verdünnung des dialysierten Solubilisierungs- bzw. Denaturierungsansatzes (siehe Beispiel 4) in Renaturierungspuffer gestartet. Die Endkonzentration der AP im Renaturierungsansatz betrug ≤ 10 µg/ml. Alle Renaturierungsansätze enthielten ein Gemisch aus reduziertem (GSH) und oxidiertem Glutathion (GSSG) um die korrekte Ausbildung der Disulfidbrücken zu erleichtern. Falls nicht anders angegeben, waren die Renaturierungsansätze bei 20 °C thermostatisiert.

Für die Renaturierung wurde die aus den Beispielen 1 bis 4 erhaltene PLAP bzw. eine kommerziell erhältliche AP aus Kälberdarm, die gemäß Beispiel 4, Solubilisierung, vorbehandelt wurde, verwendet. Da bei beiden Enzymen im wesentlichen die gleichen Ergebnisse erzielt wurden, werden im folgenden stellvertretend die mit PLAP erzielten Ergebnisse aufgezeigt.

### Nachweis der Renaturierung

Die Renaturierung der Alkalischen Phosphatase wurde über ihre Aktivität in einem Routine-Enzymtest bestimmt, bei dem die Spaltung von p-Nitrophenylphosphat spektrometrisch verfolgt wird (Bretaudiere, J.-P. and Spillman, T. (1984), Methods of Enzymatic Analysis, VCH, 75-82).

### Beispiel 6

### Versuche zur Renaturierung unter Standardbedingungen

Die Renaturierung der AP ist nicht möglich, wenn sich die Renaturierungspuffer (Standard-Puffer, siehe unten) an den in der Literatur beschriebenen Vorschlägen (Rudolph, R. (1990), Modern Methods in Protein- and Nucleic Acid Research, Walter de Gruyter, Berlin, N.Y., S. 149-171) orientieren.
Standard-Puffer:
- 0,1 mol/l: Tris-HCl/pH 8,0
- 10 mmol/l: MgCl₂
- 0,1 mmol/l: ZnCl₂
- 5 mmol/l: GSH bzw. 4 mmol/l GSH
- 1 mmol/l: GSSG bzw. 2 mmol/l GSSG

### Beispiel 7

### Renaturierung durch Na₂SO₄

Solubilisierte PLAP wurde in Renaturierungspuffer, der steigende Mengen Na₂SO₄ enthielt, verdünnt. Wie aus Tabelle 1 ersichtlich ist, kann die PLAP ab einer Na₂SO₄-Konzentration von 0,3 mol/l im Renaturierungsansatz erfolgreich renaturiert werden, wobei mit zunehmender Na₂SO₄-Konzentration auch die Renaturierungseffizienz ansteigt.
Renaturierungspuffer:
- 0,1 - 0,6 mol/l: Na₂SO₄
- 0,2 mol/l: Tris-HCl/pH 8,0
- 10 mmol/l: MgCl₂
- 0,1 mmol/l: ZnCl₂
- 4 mmol/l: GSH
- 2 mmol/l: GSSG
- Renaturierungsdauer:: 20 h bzw. 40 h

### Beispiel 8

### Renaturierung durch K₂SO₄ bzw. (NH₄)₂SO₄

Solubilisierte PLAP wurde in Renaturierungspuffer, der steigende Mengen an K₂SO₄ bzw. (NH₄)₂SO₄ enthielt, verdünnt. Beide Komponenten ermöglichen in Abhängigkeit von ihrer Konzentration eine effiziente Renaturierung der PLAP (siehe Tabelle 2).
Renaturierungspuffer:
- 0 - 1,4 mol/l: (NH₄)₂SO₄ oder
- 0 - 0,6 mol/l: K₂SO₄
- 0,2 mol/l: Tris-HCl/pH 8,0
- 10 mmol/l: MgCl₂
- 0,1 mmol/l: ZnCl₂
- 4 mmol/l: GSH
- 2 mmol/l: GSSG
- Renaturierungsdauer:: ca. 90 h

### Beispiel 9

### Renaturierung durch Kohlenhydrate oder Polyalkohole

Die Gegenwart von Zuckern bzw. Polyalkoholen und verwandten Stoffen im Renaturierungsansatz ermöglicht eine Renaturierung der PLAP. Die Abhängigkeit der Renaturierungseffizienz von der Konzentration der einzelnen Kohlenhydrate wurde systematisch untersucht. Diese Ergebnisse sind in Tabelle 3 zusammengefaßt. Daraus ist eine gesteigerte Renaturierungseffizienz in Gegenwart der untersuchten Substanzen Galactose, Glucose, Fructose, Lactose, Maltose, Saccharose, Sorbitol, Glycerin, Ethylenglykol und Arabinose ersichtlich. Weiterhin wurde eine gesteigerte Renaturierungseffizienz für Erythritol und Inositol gefunden (ohne Abbildung).
Renaturierungspuffer:
- 0 - 40 % (w/v): D(+)-Galactose, D(+)-Glucose, D(-)-Fructose, β-Lactose, Maltose-Monohydrat, Saccharose, Sorbitol, Glycerin, Ethylenglykol bzw. L(+)-Arabinose
- 0,2 mol/l: Tris-HCl/pH 8,0
- 40 mmol/l: MgCl₂
- 0,1 mmol/l: ZnCl₂
- 4 mmol/l: GSH
- 4 mmol/l: GSSG
- Renaturierungsdauer:: ca. 40 h

### Beispiel 10

### Synergistischer Effekt bei Kombination von Kohlenhydraten oder/und Polyalkoholen mit Sulfatsalzen

Der kombinatorische Effekt von Kohlenhydraten/Polyalkoholen bzw. Sulfatsalzen auf die Renaturierungseffizienz ist synergistisch. Die Gegenwart je eines Vertreters beider Stoffklassen im Renaturierungspuffer bewirkt eine höhere Renaturierungseffizienz als die von jeder Stoffklasse alleine bewirkte. In Tabelle 4 sind diese Ergebnisse für K₂SO₄ und Glycerin zusammengefaßt.
Renaturierungspuffer:
- 0 - 30 % (w/v): Glycerin in Gegenwart von 0 mol/l, 0,09 mol/l bzw. 0,18 mol/l K₂SO₄
- 0,2 mol/l: Tris-HCl/pH 8,0
- 40 mmol/l: MgCl₂
- 0,1 mmol/l: ZnCl₂
- 4 mmol/l: GSH
- 4 mmol/l: GSSG
- Renaturierungsdauer:: ca. 40 h

### Beispiel 11

### Variation des pH-Wertes im Renaturierungsansatz

In Tabelle 5 sind die Ergebnisse einer systematischen Variation des pH-Wertes vom Renaturierungspuffer dargestellt. Für alle getesten Na₂ SO₄ -Konzentrationen (0,1 bis 0,6 mol/l; Daten für 0,1 und 0,2 mol/l nicht gezeigt) liegt der optimale pH-Wert für die Renaturierung im Bereich von 8,0.
Renaturierungspuffer:
- 0,1 - 0,6 mol/l: Na₂SO₄
- 0,2 mol/l: Tris-HCl, pH 7,25-8,5
- 10 mmol/l: MgCl₂
- 0,1 mmol/l: ZnCl₂
- 4 mmol/l: GSH
- 2 mmol/l: GSSG
- Renaturierungsdauer:: ≥ 40 h

### Beispiel 12

### Variation der Redoxbedingungen im Renaturierungsansatz

Die Tabellen 6 - 8 zeigen die Abhängigkeit der Renaturierungseffizienz von der GSH- bzw. GSSG-Konzentration im Renaturierungspuffer.
Renaturierungspuffer:
- 0,2 mol/l: Tris-HCl/pH 8,0
- 10 mmol/l: MgCl₂
- 0,1 mmol/l: ZnCl₂
- 0,6 mol/l: Na₂ SO₄
- Renaturierungsdauer:: 20 h bzw. 90 h

Tabelle 6 zeigt die Renaturierungseffizienz bei Variation der GSH-Konzentration und konstanter GSSG-Konzentration (4 mmol/l GSSG). Tabelle 7 zeigt die Renaturierungseffizienz bei Variation der GSSG-Konzentration und konstanter GSH-Konzentration (4 mmol/l GSH). Tabelle 8 zeigt die Renaturierungseffizienz bei Variation der GSSG- und GSH-Konzentration und äquimolaren Mengen an GSH und GSSG im Renaturierungspuffer.

### Beispiel 13

### Variation der ZnCl₂- und MgCl₂-Konzentration im Renaturierungsansatz

Wie aus den Tabellen 9 und 10 ersichtlich ist, wird die Renaturierung der PLAP durch Zn²⁺-Ionen (z.B. ZnCl₂ ) und Mg²⁺-Ionen (z.B. MgCl₂) deutlich verbessert.

Tabelle 9 zeigt die Renaturierungseffizienz bei Variation der ZnCl₂ -Konzentration
Renaturierungspuffer:
- 0 - 0,5 mmol/l: ZnCl₂
- 0,2 mol/l: Tris-HCl/pH 8,0
- 10 mmol/l: MgCl₂
- 4 mmol/l: GSH
- 4 mmol/l: GSSG
- 0,6 mol/l: Na₂SO₄
- Renaturierungsdauer:: 20 h bzw. 90 h

Tabelle 10 zeigt die Renaturierungseffizienz bei Variation der MgCl₂ -Konzentration
Renaturierungspuffer:
- 0 - 180 mmol/l: MgCl₂
- 0,2 mol/l: Tris-HCl/pH 8,0
- 0,1 mmol/l: ZnCl₂
- 4 mmol/l: GSH
- 4 mmol/l: GSSG
- 0,6 mol/l: Na₂ SO₄
- Renaturierungsdauer:: 20 h bzw. 90 h

### Beispiel 14

### Einfluß der Temperatur auf die Renaturierungseffizienz

Die Abhängigkeit der Renaturierungseffizienz von der Renaturierungstemperatur wurde für mehrere der identifizierten Faltungsaktivatoren untersucht. Eine effiziente Renaturierung fand im gesamten getesteten Bereich von 15°C bis 30°C statt. In Tabelle 11 sind die Ergebnisse für einige der eingesetzten Faltungsaktivatoren zusammengefaßt (Na₂ SO₄ , K₂ SO₄ , (NH₄)₂SO₄, Sorbitol und Glycerin).

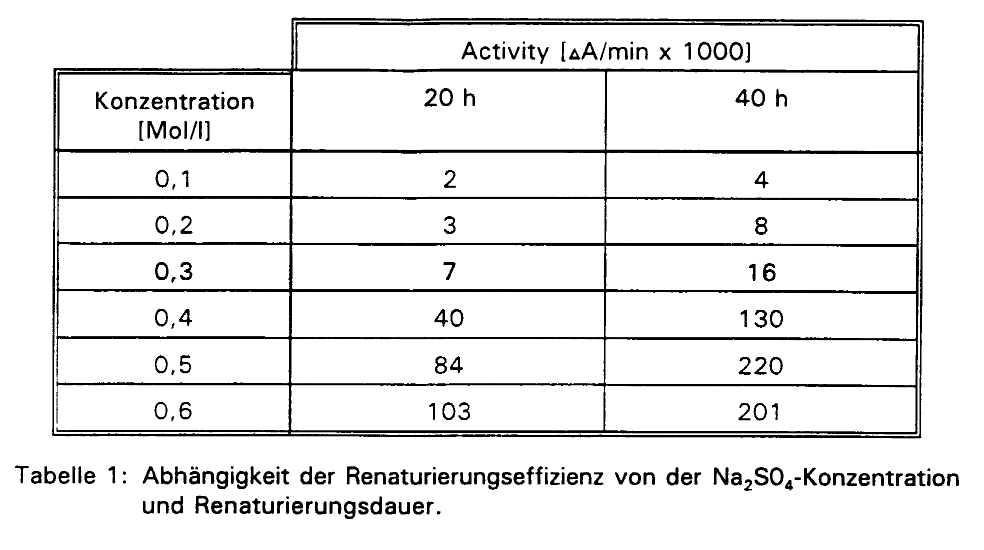

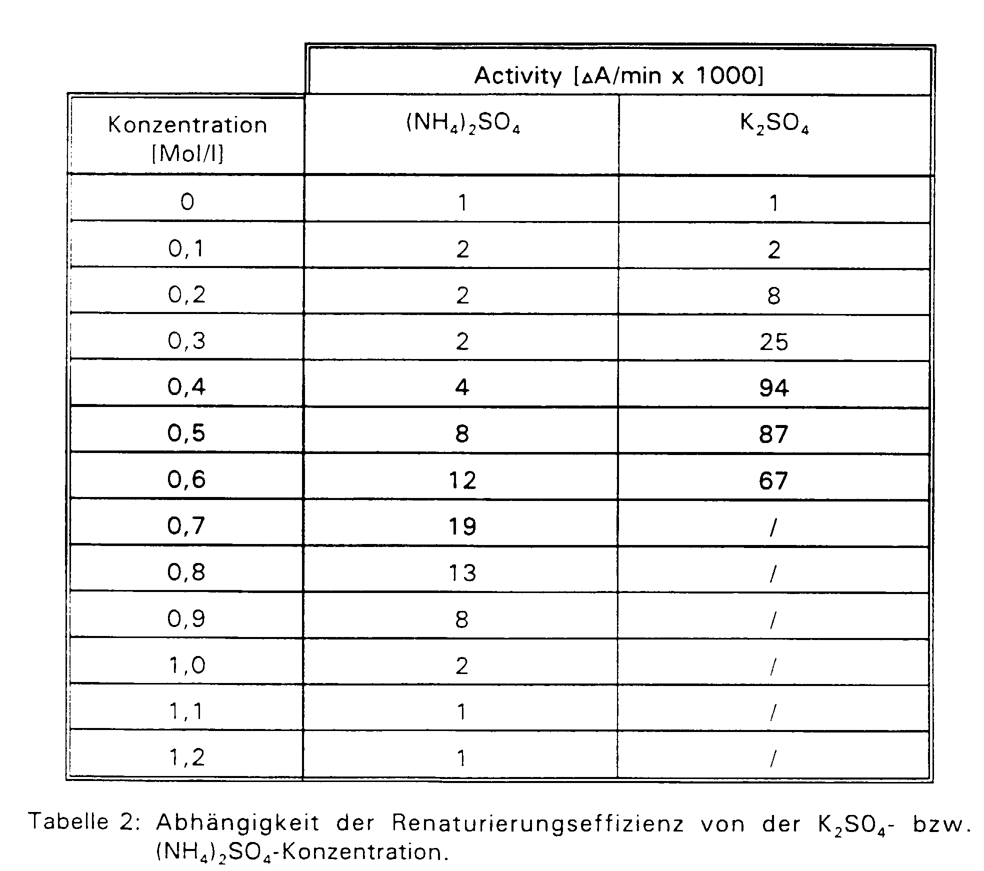

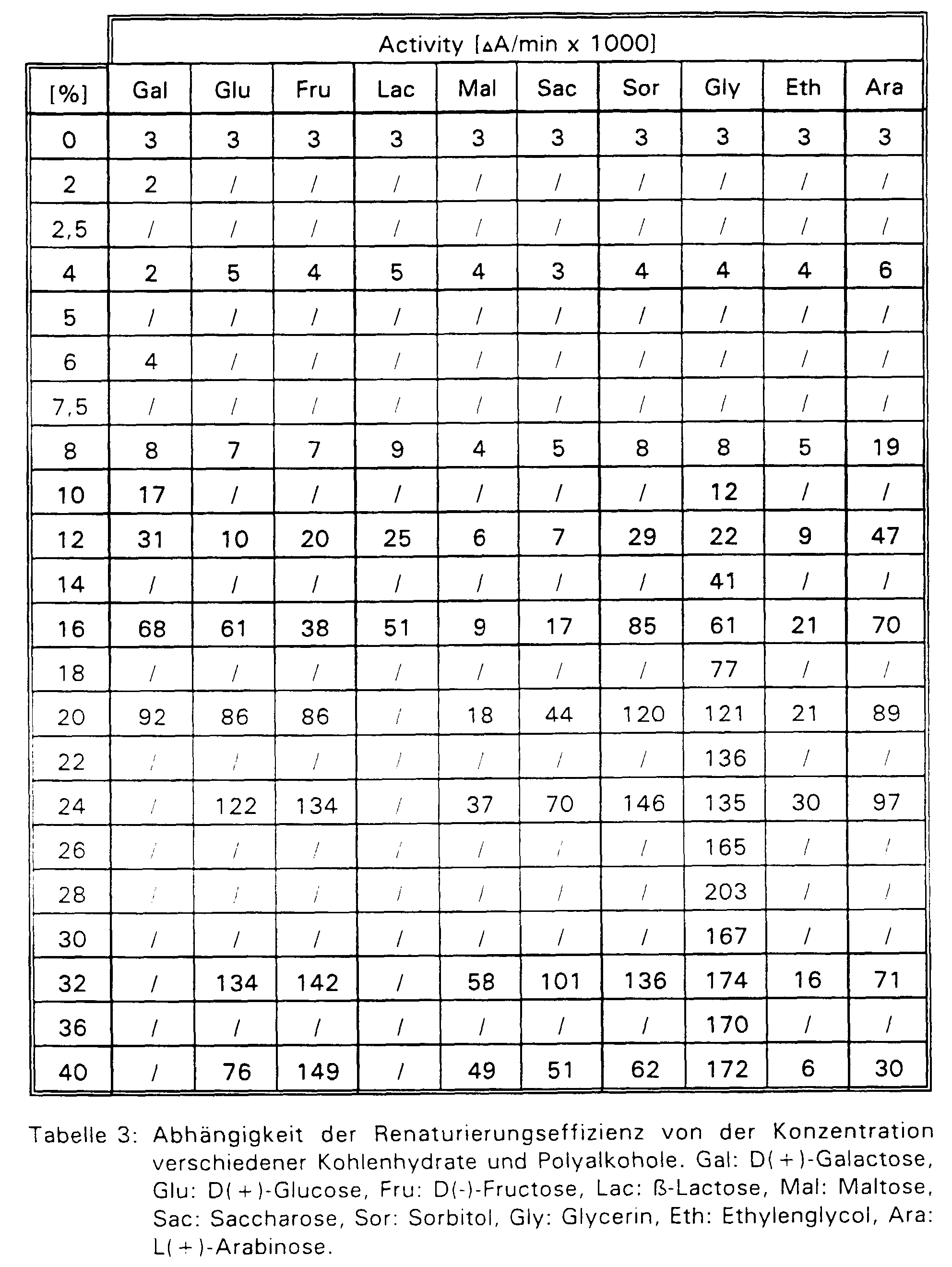

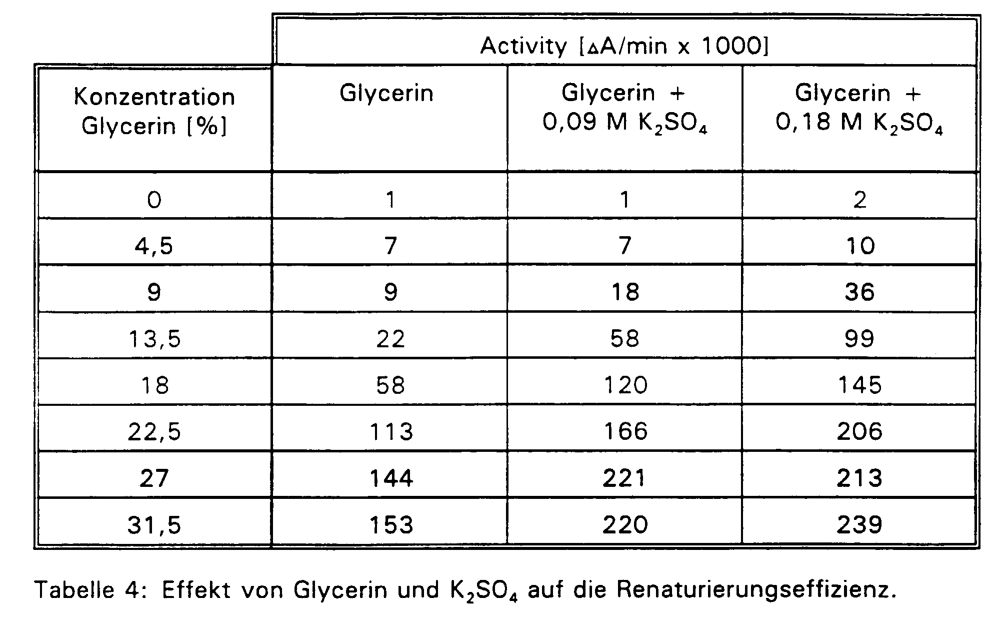

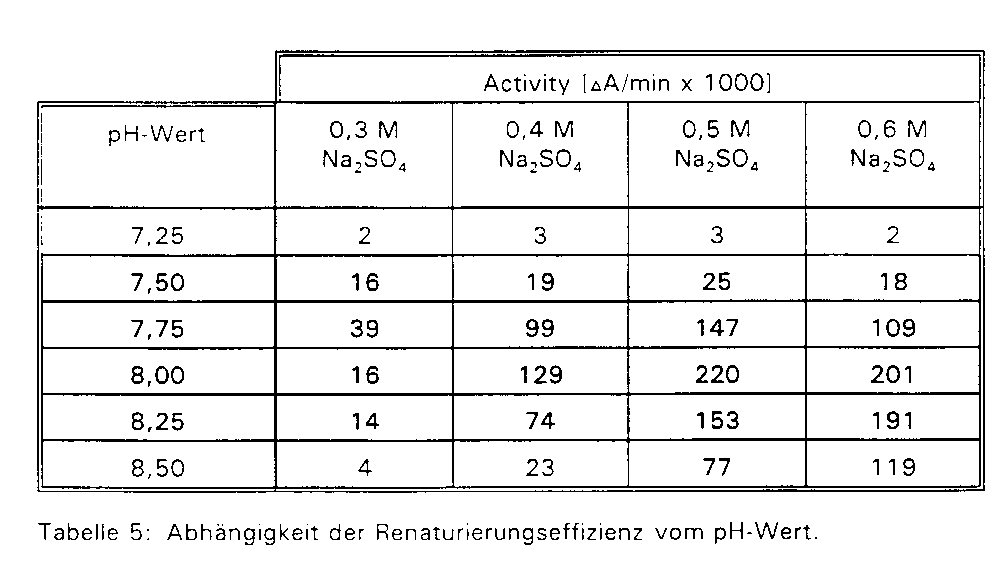

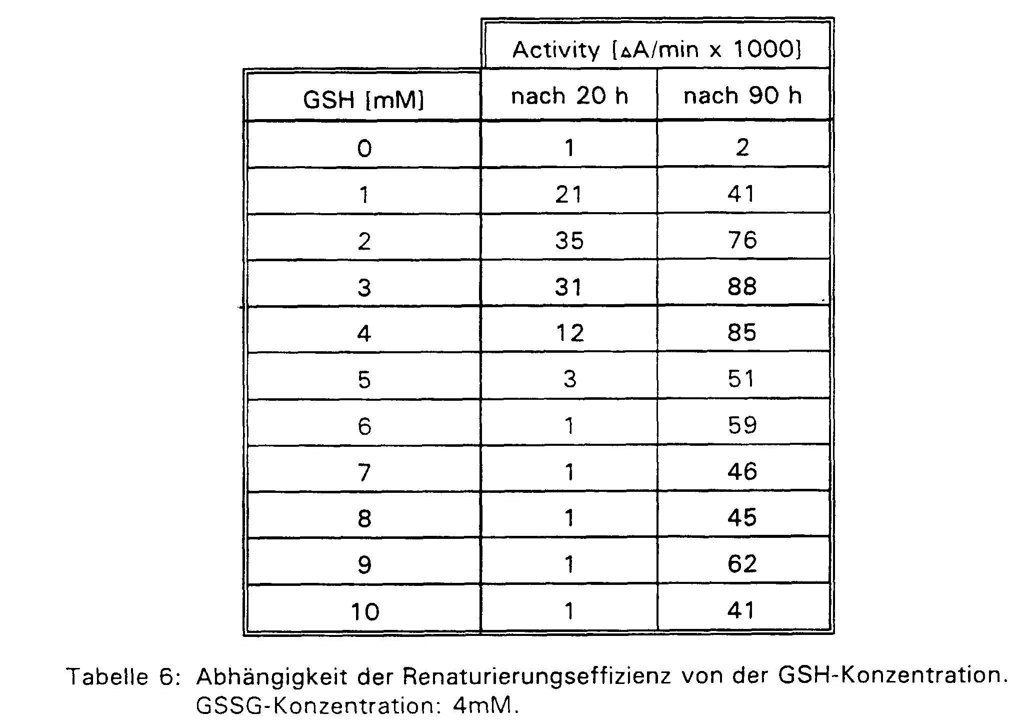

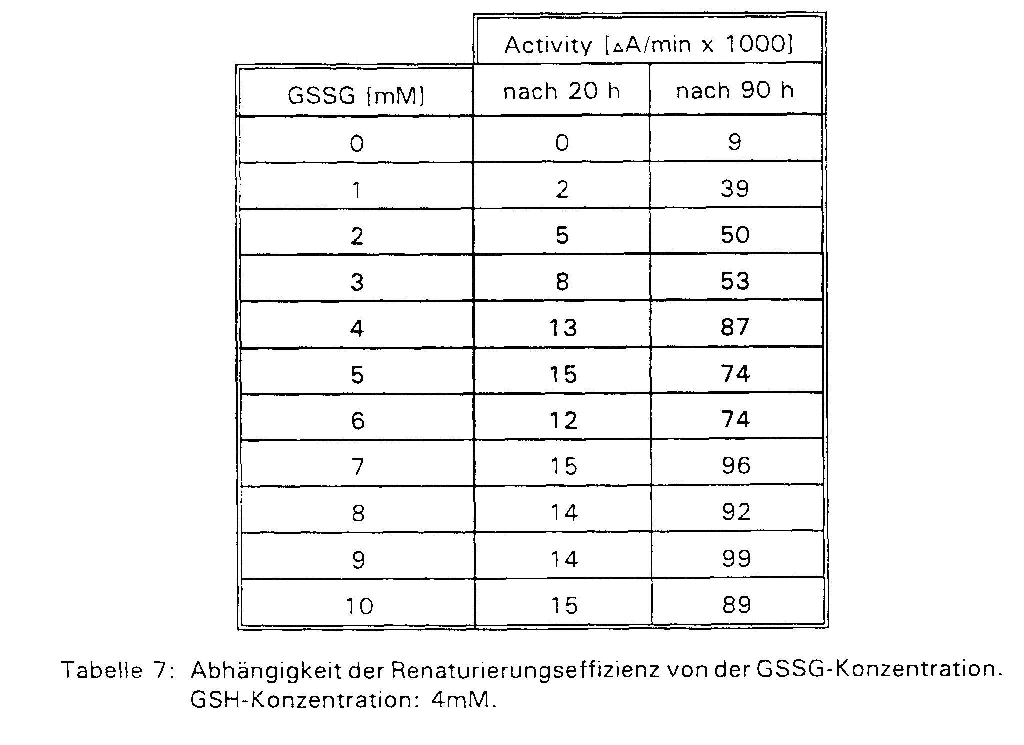

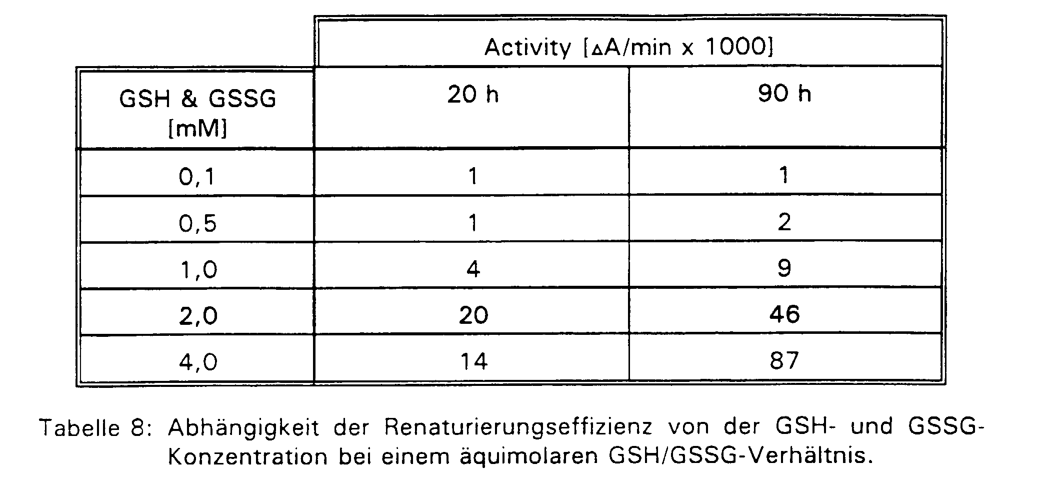

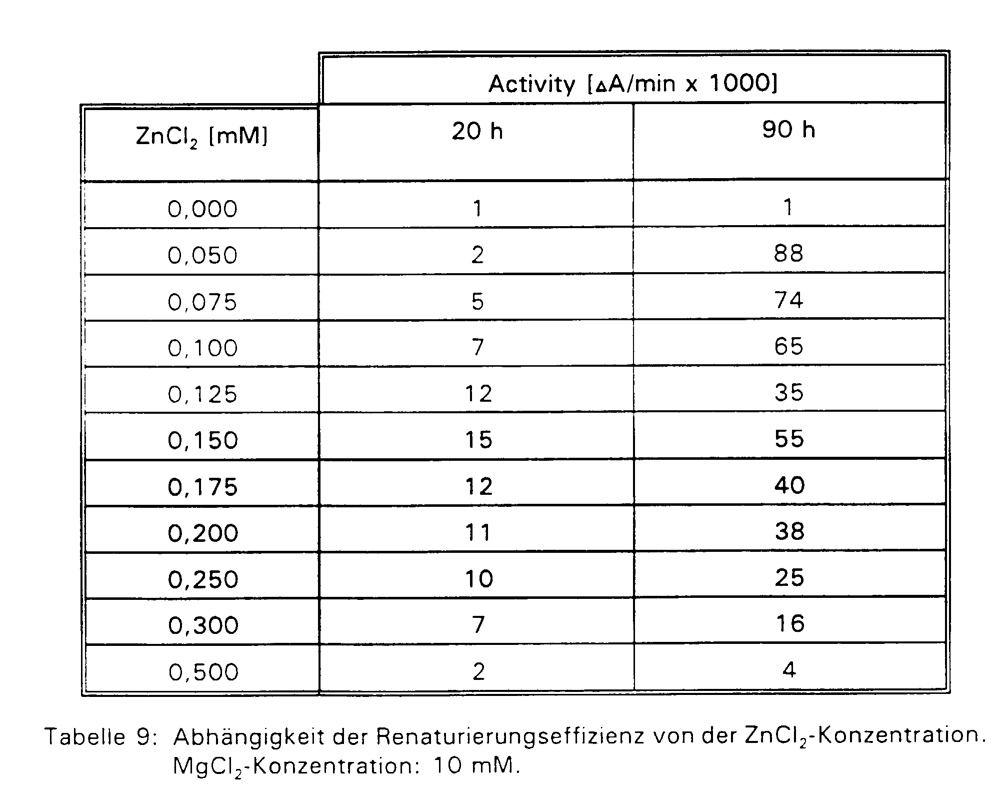

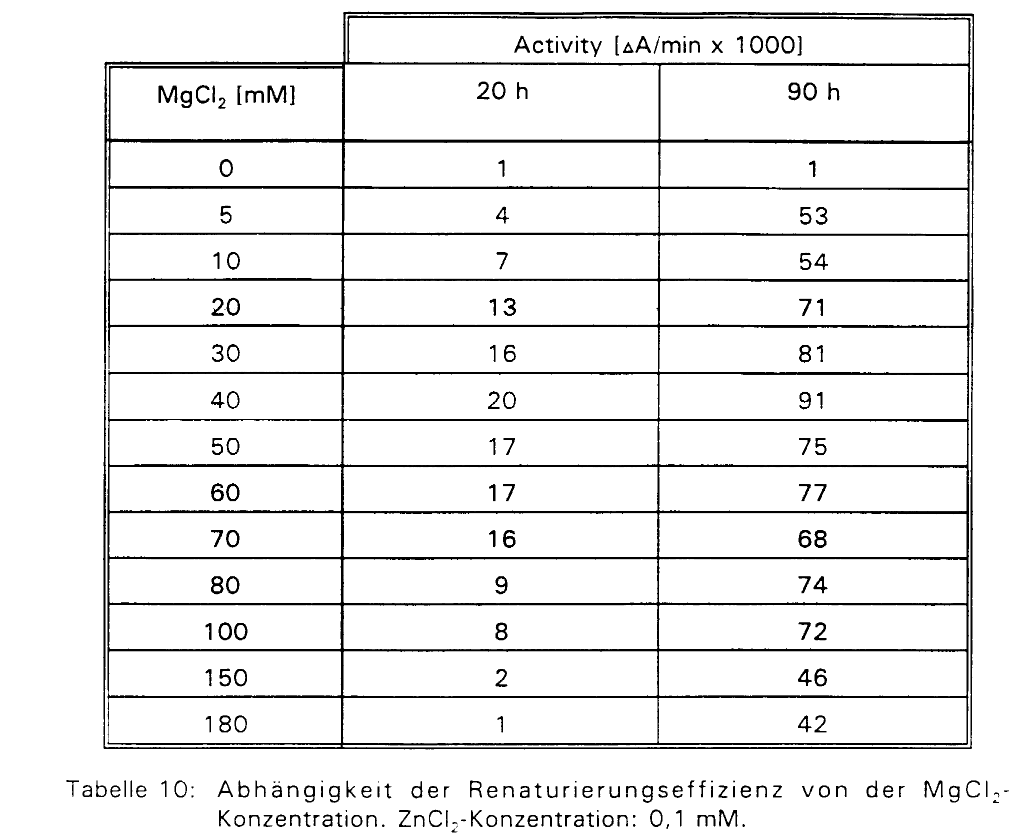

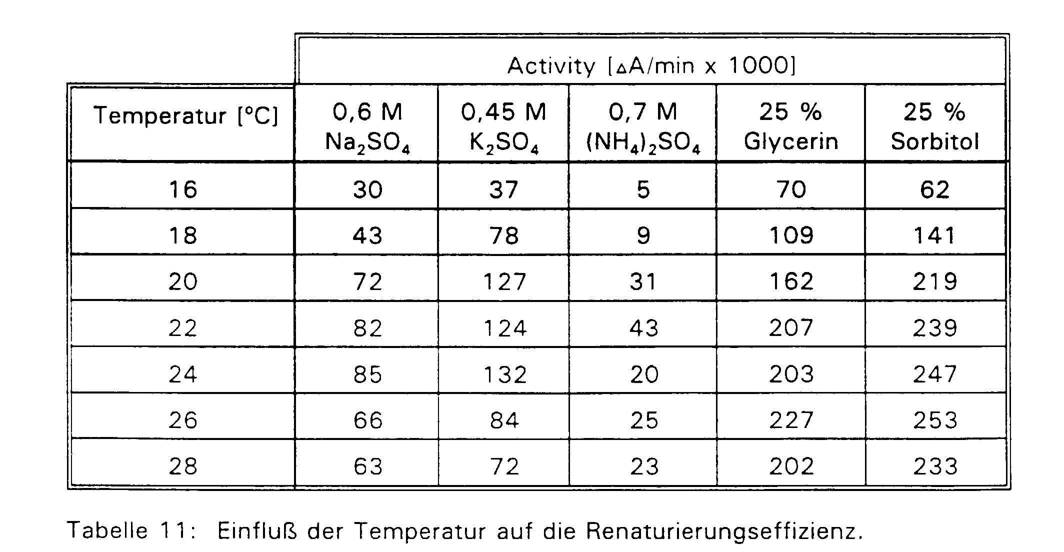

## Patentansprüche

1. Verfahren zur Gewinnung rekombinanter, biologisch aktiver, eukaryontischer alkalischer Phosphatase, bei dem eine für eine eukaryontische alkalische Phosphatase codierende DNA-Sequenz in einer prokaryontischen Wirtszelle exprimiert wird und das Expressionsprodukt durch Zellaufschluß, Solubilisierung unter denaturierenden Bedingungen und anschließender Renaturierung gewonnen wird,
**dadurch gekennzeichnet**,
daß der Renaturierungsschritt in Gegenwart eines oder mehrerer Stabilisierungsmittel durchgeführt wird, die aus synthetischen Polymeren, Mono-, Oligo- und Polysacchariden, Polyalkoholen und Salzen ausgewählt werden und in einer die Renaturierungseffizienz verbessernden Konzentration vorliegen.

2. Verfahren nach Anspruch 1,
**dadurch gekennzeichnet**,
daß als Stabilisierungsmittel Sulfatsalze verwendet werden.

3. Verfahren nach Anspruch 2,
**dadurch gekennzeichnet**,
daß Natriumsulfat in einer Menge von 0,3 - 1 mol/l, Kaliumsulfat in einer Menge von 0,1 - 0,6 mol/l und Ammoniumsulfat in einer Menge von 0,3 - 1 mol/l verwendet werden.

4. Verfahren nach Anspruch 1,
**dadurch gekennzeichnet**,
daß als Stabilisierungsmittel Kohlenhydrate verwendet werden.

5. Verfahren nach Anspruch 4,
**dadurch gekennzeichnet**,
daß die Kohlenhydrate in Mengen von 5 bis 50 Gew./Vol.-%, bezogen auf das Volumen des Renaturierungsansatzes, eingesetzt werden.

6. Verfahren nach Anspruch 1,
**dadurch gekennzeichnet**,
daß als Stabilisierungsmittel Polyalkohole mit 2 oder mehr Kohlenstoffatomen verwendet werden.

7. Verfahren nach Anspruch 6,
**dadurch gekennzeichnet,**
daß Polyalkohole in Mengen von 5 bis 50 Gew./Vol.-%, bezogen auf das Volumen des Renaturierungsansatzes, eingesetzt werden.

8. Verfahren nach einem der vorhergehenden Ansprüche,
**dadurch gekennzeichnet**,
daß als Stabilisierungsmittel Gemische von Sulfatsalzen, Kohlenhydraten oder/und Polyalkoholen verwendet werden.

9. Verfahren nach einem der vorhergehenden Ansprüche,
**dadurch gekennzeichnet**,
daß der Renaturierungsschritt in Gegenwart von Zinkionen und Magnesiumionen durchgeführt wird.

10. Verfahren nach einem der vorhergehenden Ansprüche,
**dadurch gekennzeichnet**,
daß der Renaturierungsschritt in Gegenwart von Sulfhydrylreagenzien durchgeführt wird.

11. Verfahren nach Anspruch 1,
**dadurch gekennzeichnet**,
daß die für eine eukaryontische alkalische Phosphatase codierende DNA-Sequenz auf dem Plasmid pPLAP (DSM 7094) lokalisiert ist.

12. Rekombinante, eukaryontische, alkalische Phosphatase aus einer prokaryontischen Wirtszelle, erhältlich gemäß dem Verfahren nach einem der Ansprüche 1 bis 11.

## Claims

1. Process for the production of recombinant, biologically active, eukaryotic alkaline phosphatase in which a DNA sequence coding for a eukaryotic alkaline phosphatase is expressed in a prokaryotic host cell and the expression product is isolated by cell lysis, solubilization under denaturing conditions and subsequent renaturation,
**wherein**
the renaturation step is carried out in the presence of one or several stabilizing agents which are selected from synthetic polymers, monosaccharides, oligosaccharides and polysaccharides, polyalcohols and salts and are present at a concentration which improves the renaturation efficiency.

2. Process as claimed in claim 1,
**wherein**
sulfate salts are used as stabilizing agents.

3. Process as claimed in claim 2,
**wherein**
sodium sulfate is used in an amount of 0.3 - 1 mol/l, potassium sulfate in an amount of 0.1 - 0.6 mol/l and ammonium sulfate in an amount of 0.3 - 1 mol/l.

4. Process as claimed in claim 1,
**wherein**
carbohydrates are used as stabilizing agents.

5. Process as claimed in claim 4,
**wherein**
the carbohydrates are used in amounts of 5 to 50 % w/v relative to the volume of the renaturation mixture.

6. Process as claimed in claim 1,
**wherein**
polyalcohols with 2 or more carbon atoms are used as stabilizing agents.

7. Process as claimed in claim 6,
**wherein**
polyalcohols are used in amounts of 5 to 50 % w/v relative to the volume of the renaturation mixture.

8. Process as claimed in one of the previous claims,
**wherein**
mixtures of sulfate salts, carbohydrates or/and polyalcohols are used as stabilizing agents.

9. Process as claimed in one of the previous claims,
**wherein**
the renaturation step is carried out in the presence of zinc ions and magnesium ions.

10. Process as claimed in one of the previous claims,
**wherein**
the renaturation step is carried out in the presence of sulfhydryl reagents.

11. Process as claimed in claim 1,
**wherein**
the DNA sequence coding for a eukaryotic alkaline phosphatase is present on the plasmid pPLAP (DSM 7094).

12. Recombinant eukaryotic alkaline phosphatase from a prokaryotic host cell, obtainable according to the process as claimed in one of the claims 1 to 11.

## Revendications

1. Procédé d'obtention de phosphatase alcaline eucaryote recombinée biologiquement active dans lequel une séquence d'ADN codant une phosphatase alcaline eucaryote est exprimée dans une cellule hôte procaryote et le produit d'expression est obtenu par lyse des cellules, solubilisation dans des conditions dénaturantes puis renaturation, caractérisé en ce que l'étape de renaturation est réalisée en présence d'un ou plusieurs stabilisants qui sont choisis parmi les polymères synthétiques, les mono-, oligo- et polysaccharides, les polyalcools et les sels et qui sont présents en une concentration améliorant l'efficacité de renaturation.

2. Procédé selon la revendication 1, caractérisé en ce que des sels sulfates sont utilisés comme stabilisants.

3. Procédé selon la revendication 2, caractérisé en ce que du sulfate de sodium est utilisé en une quantité de 0,3 - 1 mol/l, du sulfate de potassium est utilisé en une quantité de 0,1 - 0,6 mol/l et du sulfate d'ammonium est utilisé en une quantité de 0,3 - 1 mol/l.

4. Procédé selon la revendication 1, caractérisé en ce que des glucides sont utilisés comme stabilisants.

5. Procédé selon la revendication 4, caractérisé en ce que les glucides sont utilisés en des quantités de 5 à 50% en masse/volume par rapport au volume de la charge de renaturation.

6. Procédé selon la revendication 1, caractérisé en ce que des polyalcools ayant 2 atomes de carbone ou plus sont utilisés comme stabilisants.

7. Procédé selon la revendication 6, caractérisé en ce que les polyalcools sont utilisés en des quantités de 5 à 50% en masse/volume par rapport au volume de la charge de renaturation.

8. Procédé selon l'une des revendications précédentes, caractérisé en ce que des mélanges de sels sulfates, de glucides et/ou de polyalcools sont utilisés comme stabilisants.

9. Procédé selon l'une des revendications précédentes, caractérisé en ce que l'étape de renaturation est réalisée en présence d'ions zinc et d'ions magnésium.

10. Procédé selon l'une des revendications précédentes, caractérisé en ce que l'étape de renaturation est réalisée en présence de réactifs sulfhydrylés.

11. Procédé selon la revendication 1, caractérisé en ce que la séquence d'ADN codant une phosphatase alcaline eucaryote est localisée sur le plasmide pPLAP (DSM 7094).

12. Phosphatase alcaline eucaryote recombinée provenant d'une cellule hôte procaryote, qui peut être obtenue par le procédé selon l'une des revendications 1 à 11.
